# EUROPÄISCHE PATENTSCHRIFT

(11) **EP 3 298 346 B1**
(45) Veröffentlichungstag und Bekanntmachung des Hinweises auf die Patenterteilung: **31.07.2024**
(21) Anmeldenummer: 16730261.1
(22) Anmeldetag: 23.05.2016
(51) Int. Cl.: G01B 11/25

(54) **VORRICHTUNG ZUR OPTISCHEN 3D-VERMESSUNG EINES OBJEKTS**
DEVICE FOR OPTICAL 3D MEASURING OF AN OBJECT
DISPOSITIF POUR RÉALISER UNE MESURE EN 3D D'UN OBJET

(30) Priorität: 22.05.2015 DE 102015209402
(43) Veröffentlichungstag der Anmeldung: 28.03.2018
(73) Patentinhaber: Sirona Dental Systems GmbH, 64625 Bensheim (DE)
(72) Erfinder: THIEL, Frank, 64372 Ober-Ramstadt (DE); KOCHERSCHEIDT, Gerrit, 69190 Walldorf (DE)
(74) Vertreter: Özer, Alpdeniz
(86) Internationale Anmeldenummer: PCT/EP2016/061533
(87) Internationale Veröffentlichungsnummer: WO 2016/188939

(56) Entgegenhaltungen:
- EP-B1- 2 051 042
- WO-A1-2010/145669
- WO-A1-92/14118
- WO-A2-2008/125605

## Beschreibung

### Technisches Gebiet

Die Erfindung betrifft eine Vorrichtung zur optischen 3D-Vermessung eines Objekts unter Verwendung einer optischen tiefenscannenden Messmethode, umfassend mindestens zwei Lichtquellen, mindestens zwei optische Mittel zur Erzeugung von strukturierten Mustern und mindestens ein Aufnahmemittel, wobei mittels eines ersten optischen Mittels ein erstes Muster erzeugt wird und als ein erster Projektionsstrahl auf das aufzunehmende Objekt projiziert wird, wobei mittels eines zweiten optischen Mittels ein zweites Muster erzeugt wird und auf das aufzunehmende Objekt als ein zweiter Projektionsstrahl projiziert wird, wobei das erste Muster und das zweite Muster vom Objekt als Beobachtungsstrahlen zurückgestrahlt werden und mittels des Aufnahmemittels aufgenommen werden, um einen 3D- Datensatz des Objekts zu erzeugen.

### Stand der Technik

Aus dem Stand der Technik sind mehrere Vorrichtungen zur optischen 3D-Vermessung bekannt.

WO 2008/125605 A2 offenbart ein Verfahren und eine Anordnung zur optischen Abbildung von Objekten, insbesondere im Bereich der Mikroskopie, wobei eine erste Beleuchtungsverteilung und eine zweite Beleuchtungsverteilung auf das Objekt projiziert werden. Die beiden projizierten Beleuchtungsverteilungen können sich in ihrer Phase um 180° unterscheiden. Die Beleuchtungsstrukturen werden gleichzeitig auf das Objekt projiziert und sind durch Polarisation oder durch spektrale Charakteristik charakterisiert.

In der EP 2 051 042 B1 ist ein optisches Kohärenzverfahren offenbart, bei dem mindestens zwei Muster aus parallelen Streifen überlagert werden und dadurch ein Moiree-Muster erzeugt wird, dass auf die Oberfläche des Objekts projiziert wird. Aufgrund der Scanpositionierung der Moiree-Linien wird die dreidimensionale Form des Objekts ermittelt.

In der WO 2010/145669 A1 ein konfokales Vermessungsverfahren offenbart, bei dem ein schachbrettförmiges Projektionsmuster auf das Objekt projiziert wird. Der zeitliche Verlauf des Kontrastes wird ebenfalls ermittelt, um Tiefeninformationen des Objekts zu erhalten. Das zeitlich variierte Muster wird unter Verwendung von mechanisch angetriebenen Blendenmitteln, wie eines Blendenrads, erzeugt.

WO92/14118 A1 offenbart einen optischen Sensor und insbesondere einen Sensor, der dazu dient, den Bereich eines oder mehrerer Teile eines Objekts zu erfassen, um ein Bild davon zu erzeugen.

Ein Nachteil dieses Verfahrens besteht darin, dass das zeitlich variierte Muster unter Verwendung des mechanisch angetriebenen Blendenmittels erzeugt wird und dadurch Messfehler entstehen können.

Die Aufgabe der vorliegenden Erfindung besteht daher darin eine Vorrichtung bereitzustellen, die eine zuverlässige und präzise Vermessung des Objekts ermöglicht.

### Darstellung der Erfindung

Die Erfindung ist in den beiliegenden Ansprüchen definiert. Die betrifft eine Vorrichtung zur optischen 3D-Vermessung eines Objekts unter Verwendung einer optischen tiefenscannenden Messmethode, umfassend mindestens zwei Lichtquellen, mindestens zwei optische Mittel zur Erzeugung von strukturierten Mustern und mindestens ein Aufnahmemittel, wobei mittels eines ersten optischen Mittels ein erstes Muster erzeugt wird und als ein erster Projektionsstrahl auf das aufzunehmende Objekt projiziert wird, wobei mittels eines zweiten optischen Mittels ein zweites Muster erzeugt wird und auf das aufzunehmende Objekt als ein zweiter Projektionsstrahl projiziert wird. Dabei werden das erste Muster und das zweite Muster vom Objekt als Beobachtungsstrahlen zurückgestrahlt und mittels des Aufnahmemittels aufgenommen werden, um einen 3D- Datensatz des Objekts zu erzeugen, wobei die Vorrichtung eine Abbildungsoptik zur Projektion der Muster auf das Objekt aufweist, wobei die Abbildungsoptik so angesteuert und verstellt wird, dass eine scharfe Fokusebene schrittweise entlang einer optischen Achse der Vorrichtung verändert wird, wobei an jeder Scanposition der scharfen Fokusebene das erste Muster projiziert wird, um eine erste optische Aufnahme zu erzeugen, und anschließend zumindest das zweite Muster auf das Objekt projiziert wird, um eine zweite optische Aufnahme zu erzeugen. Die Vorrichtung ist dabei so ausgebildet, dass die erste Lichtquelle und die zweite Lichtquelle mittels einer Steuerung abwechselnd eingeschaltet werden, wobei das erste Muster und das zweite Muster abwechselnd auf das Objekt projiziert werden, wobei der erste Projektionsstrahl des ersten Musters und der zweite Projektionsstrahl des zweiten Musters mittels eines ersten Strahlteilers in eine gemeinsame Beleuchtungsrichtung zum Objekt hin umgelenkt werden.

Die Vorrichtung zur optischen 3D-Vermessung kann eine Kamera sein, die in ein herkömmliches Gehäuse in Form eines Handstücks integriert ist.

Die optische tiefenscannende Messmethode ist eine konfokale Messmethode mit dem Depth-from-defocus-Ansatz. Dabei werden abwechselnd schachbrettförmige Projektionsmuster auf das Objekt projiziert. Anschließend wird die Tiefeninformation der Messpunkte auf dem Objekt ermittelt, indem ein Wert für den Fokus beziehungsweise für die Schärfe des abgebildeten Schachbrettmusters ermittelt wird und anhand dieses Wertes der Abstand einer scharfen Fokusebene relativ zur Oberfläche des Objekts bestimmt wird.

Die Lichtquellen können beispielsweise farbige LEDs, weiße LEDs oder Laser-LEDs sein. Das optische Mittel kann ein Projektionsgitter beziehungsweise eine Projektionsmaske sein, die das Projektionsmuster erzeugt. Das optische Mittel kann auch ein digitaler Lichtprojektor aus Flüssigkeitselementen (LCD) sein, der entsprechend angesteuert wird und das Projektionsmuster erzeugt. Das Aufnahmemittel kann ein herkömmlicher optischer Sensor, wie ein CCD-Sensor sein. Das erste Muster und das zweite Muster werden also abwechselnd auf das Objekt projiziert. Die Fokussierungsoptik ist verstellbar und fokussiert die projizierten Muster auf die festgelegte scharfe Fokusebene, die schrittweise entlang der optischen Achse verändert wird, sodass das Objekt vollständig abgescannt werden kann. Die Scanpositionen können beispielsweise einen Abstand von 0,1 mm zueinander aufweisen, sodass bei 100 Scanpositionen ein Tiefenscannen eines Objektes in der Höhe von 10 mm durchgeführt werden kann. Durch diesen Abstand wird also die Auflösung entlang der optischen Achse festgelegt. Die Verstellung der Fokussierungsoptik kann dabei kontinuierlich erfolgen, wobei lediglich die Bilddaten der Aufnahmen diskret aus den festgelegten Scanpositionen ausgelesen werden.

Für jede Scanposition werden also die erste Aufnahme mit dem ersten projizierten Muster und mindestens eine zweite Aufnahme mit dem zweiten projizierten Muster erzeugt. Nach diesem Verfahren kann also die Intensität und die Intensitätsänderung für jeden Wechsel des Sensors aufgezeichnet werden. Auf diese Weise kann die Differenz der Intensitätswerte in der ersten Aufnahme und in der zweiten Aufnahme bestimmt werden und daraus der Kontrast beziehungsweise die Schärfe der beiden Aufnahmen bestimmt werden. Ausgehend von einem Kontrastwert beziehungsweise einer Schärfe kann dann ein Fokusabstand einer Oberfläche des zu vermessenden Objekts relativ zur scharfen Ebene bestimmt werden. Falls die zu vermessende Objektoberfläche von der scharfen Ebene abweicht, erscheint das Objekt in der Aufnahme unscharf. Bei bekanntem Fokusabstand der scharfen Ebene beziehungsweise der Brennweite der Fokussierungsoptik kann dann der Abstand der Objektoberfläche relativ zur Kamera berechnet werden. Zur Bestimmung der dreidimensionalen Bilddaten des Objekts nach dem Depth-from-Defocus-Messverfahren (DFD) werden die in den unterschiedlichen Scanpositionen aufgenommenen Aufnahmen miteinander verrechnet, um die dreidimensionalen Bilddaten des Objekts zu bestimmen. Dabei wird beispielsweise für jedes Pixel der Intensitätswert als Funktion einer Aufnahmenummer und damit abhängig von der Zeit sowie der Fokuslage aufgetragen. Falls das Objekt sich nicht in der Fokuslage befindet, wird der Kontrast verschlechtert. Falls das Objekt sich in der Fokuslage befindet, wird der Kontrast maximal. Die Aufnahme mit dem maximalen Kontrast wird also in der Fokuslage der Kamera aufgenommen. Auf diese Weise wird die Entfernung des Objekts relativ zur Kamera bestimmt.

Die Steuerung der Vorrichtung ist so ausgebildet, dass sie die beiden Lichtquellen mit einer hohen Frequenz abwechselnd einschaltet, sodass die beiden Muster abwechselnd auf das Objekt projiziert werden. Der Strahlteiler kann ein herkömmliches Prisma sein, das den ersten Projektionsstrahl des ersten Musters und den zweiten Projektionsstrahl des zweiten Musters aus unterschiedlichen Richtungen in eine gemeinsame Beleuchtungsrichtung umlenkt.

Ein Vorteil dieser Vorrichtung besteht darin, dass das Ändern der Muster ohne die Verwendung von mechanischen Mitteln erfolgt, sodass Messfehler verhindert werden, die durch solche mechanischen Mittel herbeigeführt werden können.

Ein weiterer Vorteil dieser Vorrichtung besteht darin, dass durch das elektronische Umschalten zwischen den beiden Lichtquellen eine viel höhere Frequenz des sich variierenden Musters erzeugt werden kann als bei der Verwendung von mechanischen Mitteln. Damit kann also die Vermessungszeit verkürzt beziehungsweise die Anzahl der Scanpositionen und damit die Auflösung des erzeugten 3D-Datensatzes des Objektes verbessert werden.

Die Vorrichtung kann beispielsweise zusätzlich zur zweiten Lichtquelle eine dritte Lichtquelle, ein drittes optisches Mittel und einen zweiten Strahlteiler aufweisen, wobei mittels des dritten optischen Mittels ein drittes Muster erzeugt wird und mittels des zweiten Strahlteilers ein dritter Projektionsstrahl in die gemeinsame Beleuchtungsrichtung umgelenkt wird.

Dadurch werden also das erste Muster, das zweite Muster und das dritte Muster abwechselnd nacheinander auf das Objekt projiziert. Durch die Verwendung mehrerer Muster kann die Auflösung in lateraler Richtung verbessert werden. Vorteilhafterweise können der erste Strahlteiler und der zweite Strahlteiler zu einem dichroitischen Prisma kombiniert sein, wobei der erste, zweite und dritte Projektionsstrahl mittels dieses dichroitischen Prismas in die gemeinsame Beleuchtungsrichtung umgelenkt werden.

Das dichroitische Prisma ist ein optisches Prisma, das einen Lichtstrahl in zwei Strahlen unterschiedlicher Spektren beziehungsweise Farben aufteilt. Es wird gewöhnlich aus Glas gefertigt, wobei bestimmte Oberflächen mit dichroitischen Spiegeln versehen sind, die Licht abhängig von dessen Wellenlänge reflektieren oder durchlassen. Unter Verwendung eines solchen dichroitischen Prismas können also drei Projektionsstrahlen aus unterschiedlichen Richtungen in eine gemeinsame Beleuchtungsrichtung umgelenkt werden. Dabei kann der erste Projektionsstrahl einen Rotanteil, der zweite Projektionsstrahl einen Grünanteil und der dritte Projektionsstrahl einen Blauanteil aufweisen. Die Erzeugung der farbigen Projektionsstrahlen kann beispielsweise durch die Verwendung von farbigen LEDs oder durch die Verwendung von Farbfiltern erfolgen, die einer Lichtquelle nachgeordnet sind.

Eine Ausführungsform mit drei Lichtquellen ist allerdings nicht Teil der beanspruchten Erfindung.

Vorteilhafterweise können die optischen Mittel zur Erzeugung der Muster optische Gitter oder Flüssigkristall-Elemente (LCD) sein.

Das optische Gitter oder der LCD erzeugen dadurch die projizierten Muster. Das LCD wird dabei entsprechend gesteuert.

Vorteilhafterweise können die projizierten Muster die Form von parallelen Streifen aufweisen.

Nach den verwendeten Tiefenscanverfahren werden also der Kontrast und damit die Tiefeninformationen im Übergangsbereich zwischen den dunklen und hellen parallelen Streifen ermittelt.

Vorteilhafterweise kann bei lediglich zwei Lichtquellen die Breite der parallelen Streifen dem Abstand zwischen den parallelen Streifen entsprechen.

Dadurch ergänzt sich das erste Muster und das zweite Muster mit den parallelen Streifen, sodass an der Scanposition eines hellen Streifens des ersten Musters ein dunkler Streifen des zweiten Musters angeordnet ist und umgekehrt.

Dadurch werden also der Kontrast und die Tiefeninformationen für die gleichen Messpunkte des Objekts ermittelt, sodass dadurch die Messgenauigkeit verbessert werden kann. Vorteilhafterweise können die projizierten Muster die Form eines Schachbretts mit quadratischen Musterelementen aufweisen.

Durch die Form eines Schachbretts der projizierten Muster werden also Kontrastwerte und damit Tiefeninformationen in den Übergangsbereichen zwischen den hellen Musterelementen und den dunklen Musterelementen ermittelt, sodass die Auflösung des 3D-Datensatzes des Objekts verbessert wird.

Vorteilhafterweise können das erste Muster ein erstes schachbrettförmiges Muster sein und das zweite Muster ein zweites schachbrettförmiges Muster sein, wobei an der Scanposition eines hellen quadratischen Musterelements des ersten schachbrettförmigen Musters ein dunkles quadratischen Musterelement des zweiten schachbrettförmigen Musters angeordnet ist und umgekehrt, so dass die hellen Musterelemente und die dunklen Musterelemente der beiden schachbrettförmigen Muster jeweils abwechselnd auf das Objekt projiziert werden.

Dadurch ergänzen sich die beiden schachbrettförmigen Muster, sodass die Kontrastwerte und damit die Tiefeninformationen für die gleichen Messpunkte auf der Objektoberfläche ermittelt werden, dadurch können mögliche Messfehler vermindert werden.

Vorteilhafterweise kann die Vorrichtung eine Steuerung aufweisen, die die Lichtquellen mit einer Frequenz von mindestens 3000 Hz abwechselnd ein- und abschaltet.

Durch die relativ hohe Frequenz der variierenden Muster kann das Objekt mittels des tiefenscannenden Verfahrens innerhalb einer kurzen Vermessungszeit vermessen werden.

Vorteilhafterweise kann die Frequenz vorteilhafterweise zwischen 5000 Herz und 10000 Herz betragen.

In einem Beispiel der Vorrichtung beträgt die Frequenz 8000 Herz. Dabei erfolgt der komplette Tiefenscan des gesamten Objekts mit 20 Herz, wobei für jede der 200 Tiefenpositionen jeweils beide Muster projiziert werden und beide Aufnahmen aufgenommen werden. Durch eine solche schnelle Vermessung kann die Vorrichtung in Form einer handgehaltenen Kamera freigehalten werden, da die Bewegungen einer handgehaltenen Kamera relativ zu den Objekten, wie den Zähnen eines Patienten oder eines Zahnmodells, bei einer kompletten Vermessung mit 20 Herz zu vernachlässigen sind.

Vorteilhafterweise können zur Erzeugung der optischen Aufnahme bzw. eines Scans mindestens 100 Scanpositionen der scharfen Fokusebene schrittweise eingestellt und vermessen werden.

Bei 100 Scanpositionen kann der Abstand zwischen den Scanpositionen beispielsweise 0,2 mm betragen, da der typische Tiefenscanbereich zur Vermessung von Zähnen 20 mm beträgt.

Vorteilhafterweise können pro Aufnahme mindestens 200 Scanpositionen der scharfen Fokusebene eingestellt werden.

Durch die höhere Anzahl von Scanpositionen wird die Auflösung entlang der optischen Achse verbessert. Beispielsweise kann der Abstand zwischen den Scanpositionen 0,1 mm betragen.

Vorteilhafterweise können Kontrastwerte zwischen den hellen und den dunklen Elementen der Muster für jede optische Aufnahme bestimmt werden, wobei anhand der Kontrastwerte 3D-Tiefeninformationen des Objekts bestimmt werden.

Nach den verwendeten Tiefenscanverfahren werden in den Übergangsbereichen zwischen den hellen und den dunkeln Elementen der Muster Kontrastwerte und daraus die Tiefeninformationen ermittelt.

Vorteilhafterweise kann für jeden Messpunkt des Objekts ein Verlauf der Kontrastwerte für alle eingestellten Scanpositionen der scharfen Ebene ermittelt werden, wobei anschließend ein Maximalwert dieses Verlaufs bestimmt wird, der die Tiefeninformation der Objektoberfläche für diesen Messpunkt des Objekts darstellt.

Dadurch werden also die Tiefeninformationen für die Messpunkte der Objektoberfläche in dem Grenzbereich zwischen den dunklen und den hellen Elementen ermittelt. Die Berechnung des 3D-Datensatzes des Objekts aus den einzelnen optischen Aufnahmen der variierten Muster kann mittels eines Computers erfolgen.

### Kurzbeschreibung der Zeichnungen

Die Erfindung wird anhand der Zeichnungen erläutert. Es zeigt, die
- Fig. 1: zeigt eine Skizze einer Vorrichtung zur optischen 3D-Vermessung eines Objekts, die
- Fig. 2: eine Skizze eines schachbrettförmigen Projektionsmusters, die
- Fig. 3: eine Skizze eines Projektionsmusters, der aus dunklen Streifen und hellen Streifen besteht, die
- Fig .4: ein Diagramm eines Intensitätswertes in Abhängigkeit von der Zeit, die
- Fig. 5: ein Diagramm eines Differenzwertes in Abhängigkeit von der Zeit, die
- Fig .6: ein Diagramm des Differenzwertes in Abhängigkeit von einem Fokusabstand.

### Ausführungsbeispiele

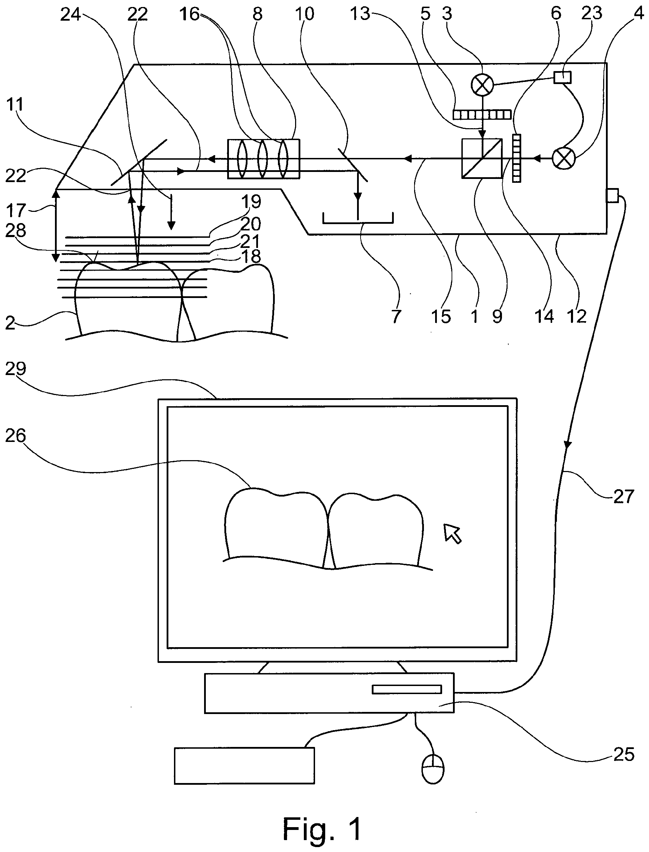

Die Fig. 1 zeigt eine Skizze einer Vorrichtung 1 zur optischen 3D-Vermessung eines Objekts 2. Die Vorrichtung 1 ist eine handgehaltene dentale Kamera zur Vermessung der Objekte 2, wie der Zähne eines Patienten oder eines Zahnmodells. Die Kamera 1 weist eine erste Lichtquelle 3, eine zweite Lichtquelle 4, ein erstes optische Mittel 5, ein zweites optische Mittel 6 zur Erzeugung von strukturierten Mustern, ein Aufnahmemittel 7, eine Abbildungsoptik 8, einen ersten Strahlteiler 9, einen zweiten Strahlteiler 10, einen Spiegel 11 und ein Gehäuse 12 auf. Die Lichtquellen 3 und 4 können als einfache LEDs, farbige LEDs, weiße LEDs oder Laser-LEDs ausgeführt sein. Die optischen Mittel 5 und 6 können als optische Gitter oder als LCDs ausgeführt sein, die entsprechend angesteuert werden, um die projizierten Muster zu erzeugen. Mittels des ersten optischen Mittels 5 wird das erste Muster projiziert, das als ein erster Projektionsstrahl 13 in die Richtung des ersten Strahlteilers 9 abgestrahlt wird. Mittels des zweiten optischen Mittels 6 wird ein zweites Muster als ein zweiter Projektionsstrahl 14 in die Richtung des ersten Strahlteilers 9 abgestrahlt. Mittels des ersten Strahlteilers 9 werden die Projektionsstrahlen 13 und 14 in einer gemeinsamen Beleuchtungsrichtung 15 zum Objekt 2 hin umgelenkt. Das Aufnahmemittel 7 ist ein optischer Sensor, wie ein CCT-Sensor. Die Abbildungsoptik 8 weist mehrere Linsen 16 auf, die so angesteuert werden, dass ein Fokusabstand 17 einer scharfen Ebene 18 relativ zur Kamera 1 schrittweise zwischen mehreren festgelegten Scanpositionen 19, 20 und 21 verstellt wird. Für jede Scanposition 18, 19, 20 und 21 wird eine erste Aufnahme mit dem ersten projizierten Muster und eine zweite Aufnahme mit dem zweiten projizierten Muster erzeugt. Das projizierte Muster kann dabei beispielsweise eine schachbrettförmige Form aufweisen oder aus mehreren bestehen. Das projizierte Muster wird also auf das Objekt 2 projiziert und als ein Beobachtungsstrahl 22 vom Objekt 2 zurückgestrahlt, mittels des zweiten Strahlteilers 10 umgelenkt und mittels des Aufnahmemittels 7 aufgenommen. Mittels einer Steuerung 23 werden die Lichtquellen 3 und 4 abwechselnd eingeschaltet, sodass das erste Muster und das zweite Muster abwechselnd auf das Objekt 2 projiziert werden. Der Schaltvorgang mittels der Steuerung 23 kann beispielsweise mit einer Frequenz von 8000 Hz erfolgen. Die Scanpositionen 18, 19, 20 und 21 können beispielsweise in einem Abstand von 0,1 mm voneinander angeordnet sein. Die Auflösung entlang einer optischen Achse 24 ist damit durch diesen Abstand zwischen den Scanpositionen gegeben. Das schachbrettförmige Muster kann aus mehreren quadratischen hellen und dunklen Musterelementen aufgebaut sein, wobei die Breite eines Musterelements in der Projektionsebene des Aufnahmemittels 7 der Breite eines Pixels des verwendeten Sensors entsprechen kann. Dadurch wird also für jeden Pixel des Sensors ein erster Intensitätswert für ein helles Musterelement und ein zweiter Intensitätswert für ein dunkles Musterelelement bestimmt, wobei mittels einer Recheneinheit 25, wie eines Computers, durch Differenzbildung ein Differenzwert zwischen dem ersten Intensitätswert und dem zweiten Intenstiätswert ermittelt werden kann. Die Recheneinheit 25 kann auch ein Mikrocomputer oder ein Chip sein, der in die Kamera 1 integriert ist. Die Recheneinheit 25 kann auch ein externer Computer sein. Die für die unterschiedlichen Scanpositionen 18, 19, 20 und 21 erzeugten optischen Aufnahmen werden nach jedem Scanvorgang verrechnet und daraus ein 3D-Datensatz 26 des Objekts 2 erzeugt. Das Verrechnen kann auch mittels eines anderen Verfahrens unter Verwendung der Recheneinheit 25 erfolgen. Dabei können beispielsweise mehrere Kontrastwerte in den Übergangsbereichen zwischen den hellen und dunklen Musterelementen ermittelt werden und daraus die Tiefeninformationen berechnet werden. Die Bilddaten des Sensors 7 werden nach jeder optischen Aufnahme ausgelesen, wie durch den Pfeil 27 dargestellt ist und an die Recheneinheit 25 übermittelt. Die ermittelten Koordinaten der Messpunkte auf einer Objektoberfläche 28 werden nach der vollständigen Vermessung des Objekts 2 verwendet, um mit den 3D-Datensatz 26 zu berechnen. Der 3D-Datensatz 26 wird mittels einer Anzeigevorrichtung 29, wie eines Monitors, dargestellt.

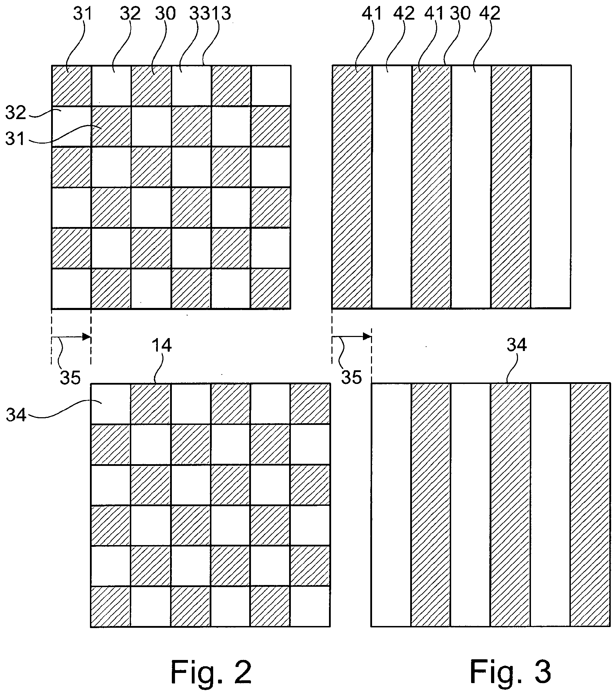

Die Fig. 2 zeigt eine Skizze eines ersten schachbrettförmigen projizierten Musters 30, das als der erste Projektionsstrahl 13 von der ersten Lichtquelle 3 abgestrahlt wird und mittels des Sensors 7 in der ersten Scanposition 18 aus Fig. 1 aufgenommen wird. Die dunklen Musterelemente 31 des ersten projizierten Musters 30 sowie die hellen Musterelemente 32 des ersten projizierten Musters 30 entsprechen in den Abmessungen den einzelnen Pixeln 33 des Sensors 7.

Durch das Abschalten der ersten Lichtquelle 3 und das Einschalten der zweiten Lichtquelle 4 wird also das zweite projizierte Muster 34 auf das Objekt 2 projiziert, das als der zweite Projektionsstrahl 14 von der zweiten Lichtquelle 3 abgestrahlt wird und mittels des Sensors 7 in der ersten Scanposition 18 aus Fig. 1 aufgenommen wird. Das erste projizierte Muster 30 entspricht dabei in der Form dem zweiten projizierten Muster 34, wobei das zweite Muster 34 seitlich verschoben ist, wobei die Verschiebung 35 der Breite eines Pixels 33 entspricht. Auf diese Weise wird also jeder Pixel des Sensors 7 abwechselnd mit einem hellen Musterelement 32 oder einem dunklen Musterelement 31 beleuchtet. Dadurch lässt sich also aus der ersten Aufnahme des ersten Musters 30 und aus der zweiten Aufnahme des zweiten Musters 34 für jedes Pixel ein Intensitätswert für jeweils ein helles und dunkles Musterelements ermitteln. Falls die scharfe Schicht mit der Objektoberfläche 28 des Objekts 2 übereinstimmt, wird das Projektionsmuster 30 scharf auf den Sensor 7 abgebildet, so dass die Differenzwerte der Intensitätswerte für die einzelnen Pixel im Maximum liegen.

Die Fig. 3 zeigt eine Skizze eines ersten projizierte Muster 30, das aus dunklen Streifen 41 und hellen Streifen 42 besteht. Das zweite projizierte Muster 34 ist im Vergleich zum ersten projizierten Muster, wie in Fig. 2, um eine Verschiebung 35 verschoben, die einer Pixelbreite des Sensors 7 entspricht. Durch das abwechselnde Einschalten der Lichtquellen 3 und 4 wird also jeder Pixel des Sensors 7 abwechselnd mit einem hellen Streifen 42 oder einem dunklen Streifen 41 beleuchtet.

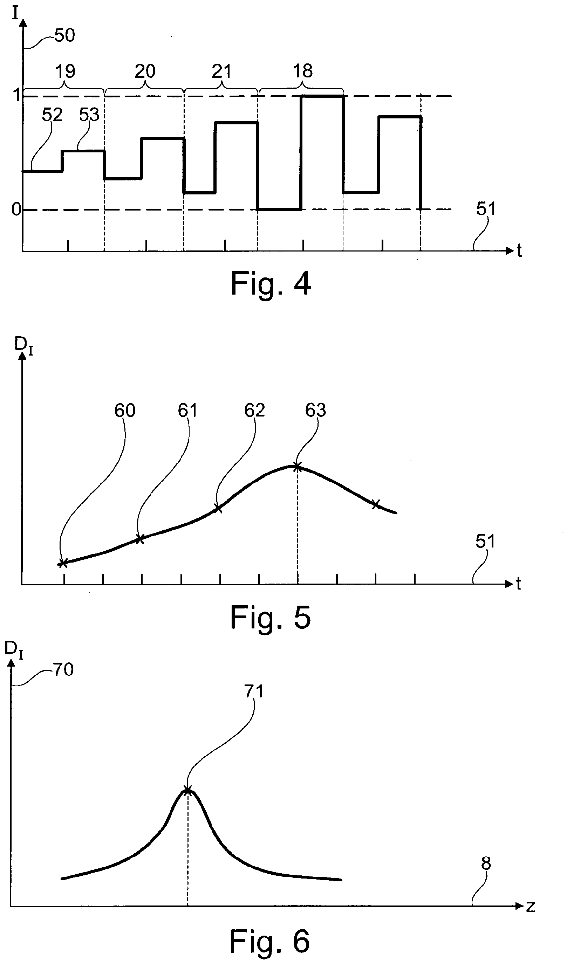

Die Fig. 4 zeigt ein Diagramm eines Intensitätswertes 50 auf der Y-Achse in Abhängigkeit von der Zeit 51 auf der X-Achse. Dabei wird bei der Beleuchtung des Objekts mit dem ersten Muster 30 ein erster Intensitätswert 52 auf einer Skala zwischen 0 und 1 aus der ersten Aufnahme und ein zweiter Intensitätswert 53 bei der Beleuchtung des Objekts mit dem zweiten Muster 34 aus der zweiten ermittelt. Die Intensitätswerte werden für die Scanpositionen 18, 19, 20 und 21 aus Fig. 1 ermittelt.

Die Fig. 5 zeigt ein Diagramm zur Verdeutlichung des vorliegenden Verfahrens, wobei aus dem Intensitätswert 52 und 53 durch Differenzbildung ein erster Differenzwert 60 für die erste Scanposition 19, ein zweiter Differenzwert 61 für die zweite Scanposition 20, ein dritter Differenzwert 62 für die dritte Scanposition 21 und ein vierter Differenzwert 63 für die vierte Scanposition 18 ermittelt werden. Die Differenzwerte sind in Abhängigkeit von der Zeit 51 aufgetragen. In der vierten Scanposition 18 ist der Differenzwert 63 maximal, so dass in dieser Scanposition 18 die scharfe Ebene 7 mit der Oberfläche 28 des Objekts übereinstimmt. Dadurch lässt sich also für jedes Pixel eine Tiefeninformation des entsprechenden Messpunktes auf der Oberfläche 28 des Objekts ermitteln.

Die Fig. 6 zeigt den Differenzwert 70 in Abhängigkeit vom Fokusabstand 17 für einen einzelnen Pixel des Sensors 7. In einem Maximum 71 des Differenzwertes ist der Kontrast maximal, da das Projektionsmuster 30, 34 aus Fig. 2 scharf abgebildet wird.

### Bezugszeichen

1 Vorrichtung
2 Objekt
3 erste Lichtquelle
4 zweite Lichtquelle
5 erstes optische Mittel
6 zweites optische Mittel
7 Aufnahmemittel
8 Abbildungsoptik
9 erster Strahlteiler
10 zweiter Strahlteiler
11 Spiegel
12 Gehäuse
13 erster Projektionsstrahl
14 zweiter Projektionsstrahl
15 Beleuchtungsrichtung
16 Linsen
17 Fokusabstand
18 scharfe Ebene
18 Scanposition
19 Scanposition
20 Scanposition
21 Scanposition
22 Beobachtungsstrahl
23 Steuerung
24 optische Achse
25 Recheneinheit
26 3D-Datensatz
27 Pfeil
28 Objektoberfläche
29 Anzeigevorrichtung
30 erstes projiziertes Muster
31 dunkle Musterelemente
32 helle Musterelemente
33 Pixel
34 zweites projiziertes Muster
35 Verschiebung
41 dunkle Streifen
42 helle Streifen
50 Intensitätswert
51 Zeit
52 erster Intensitätswert
53 zweiter Intensitätswert
60 erster Differenzwert
61 zweiter Differenzwert
62 dritter Differenzwert
63 vierter Differenzwert
70 Differenzwert

## Patentansprüche

1. Vorrichtung zur optischen 3D-Vermessung eines Objekts (2) unter Verwendung einer optischen tiefenscannenden Messmethode, umfassend mindestens zwei Lichtquellen (3, 4), mindestens zwei optische Mittel (5, 6) zur Erzeugung von strukturierten Mustern (30, 34) und mindestens ein Aufnahmemittel (7), wobei mittels eines ersten optischen Mittels (5) ein erstes Muster (30) erzeugt wird und als ein erster Projektionsstrahl (13) auf das aufzunehmende Objekt (2) projiziert wird, wobei mittels eines zweiten optischen Mittels (6) ein zweites Muster (14) erzeugt wird und auf das aufzunehmende Objekt (2) als ein zweiter Projektionsstrahl (14) projiziert wird, wobei das erste Muster (30) und das zweite Muster (34) vom Objekt (2) als Beobachtungsstrahlen (22) zurückgestrahlt werden und mittels des Aufnahmemittels (7) aufgenommen werden, um einen 3D- Datensatz des Objekts zu erzeugen, wobei die Vorrichtung (1) eine Abbildungsoptik (8) zur Projektion der Muster (30, 34) auf das Objekt (2) aufweist, wobei die Abbildungsoptik (8) so angesteuert und verstellt wird, dass eine scharfe Fokusebene (18) schrittweise entlang einer optischen Achse (24) der Vorrichtung verändert wird, wobei an jeder Scanposition (18, 19, 20, 21) der scharfen Fokusebene das erste Muster (30) projiziert wird, um eine erste optische Aufnahme zu erzeugen, und anschließend zumindest das zweite Muster (34) auf das Objekt (2) projiziert wird, um eine zweite optische Aufnahme zu erzeugen, wobei die Vorrichtung so ausgebildet ist, dass die erste Lichtquelle (3) und die zweite Lichtquelle (4) mittels einer Steuerung (23) abwechselnd eingeschaltet werden, wobei das erste Muster (30) und das zweite Muster (34) abwechselnd auf das Objekt (2) projiziert werden, wobei der erste Projektionsstrahl (13) des ersten Musters (30) und der zweite Projektionsstrahl (14) des zweiten Musters (34) mittels eines ersten Strahlteilers (9) in eine gemeinsame Beleuchtungsrichtung (15) zum Objekt (2) hin umgelenkt werden, wobei die projizierten Muster (30, 34) die Form eines Schachbretts mit quadratischen Musterelementen (31, 32) oder die Form von parallelen Streifen (41, 42) aufweisen, wobei die optische tiefenscannende Messmethode ein Depth-from-Defocus-Messverfahren, DFD, ist,
wobei helle Musterelemente (32) und dunkle Musterelemente (31) der beiden Muster (30, 34) jeweils abwechselnd auf das Objekt (2) projizierbar sind und sich ergänzen, so dass an einer Scanposition eines hellen Musterelements (32) des ersten Musters (30) ein dunkles Musterelement (31) des zweiten Musters (34) angeordnet ist und umgekehrt, wobei das optische Mittel das das Projektionsmuster erzeugt als Projektionsgitter, Projektionsmaske oder digitaler Lichtprojektor aus Flüssigkeitselementen, LCD, ausgebildet ist,
**dadurch gekennzeichnet, dass** Tiefeninformationen von Messpunkten auf dem Objekt in einem Grenzbereich zwischen den dunklen und hellen Musterelementen (32) ermittelt werden, indem Kontrastwerte des projizierten Musters (30, 34) ermittelt werden und anhand dieser Kontrastwerte der Abstand einer scharfen Fokusebene relativ zu den Messpunkten auf der Oberfläche des Objekts bestimmt wird, wobei zur Bestimmung der Kontrastwerte der Messpunkte eine Differenz von Intensitätswerten in der ersten Aufnahme und in der zweiten Aufnahme bestimmt wird.

2. Vorrichtung nach Anspruch 1, **dadurch gekennzeichnet, dass** die Vorrichtung eine Steuerung aufweist, die die Lichtquellen mit einer Frequenz von mindestens 3000 Hz abwechselnd ein- und abschaltet.

3. Vorrichtung nach Anspruch 2, **dadurch gekennzeichnet, dass** die Frequenz vorteilhafterweise zwischen 5000 Hz und 10000 Hz beträgt.

4. Vorrichtung nach einem der Ansprüche 1 bis 3, **dadurch gekennzeichnet, dass** zur Erzeugung der optischen Aufnahme bzw. eines Scans mindestens 100 Scanpositionen (18, 19, 20, 21) der scharfen Fokusebene schrittweise eingestellt und vermessen werden.

5. Vorrichtung nach Anspruch 4, **dadurch gekennzeichnet, dass** pro Aufnahme mindestens 200 Scanpositionen (18, 19, 20, 21) der scharfen Fokusebene eingestellt werden.

6. Vorrichtung nach einem der Ansprüche 1 bis 5, **dadurch gekennzeichnet, dass** Kontrastwerte zwischen den hellen und den dunklen Elementen (31, 32) der Muster (30, 34) für jede optische Aufnahme bestimmt werden, wobei anhand der Kontrastwerte (52, 53, 60, 61, 62, 63) 3D-Tiefeninformationen des Objekts (2) bestimmt werden.

7. Vorrichtung nach Anspruch 6, **dadurch gekennzeichnet, dass** für jeden Messpunkt des Objekts ein Verlauf der Kontrastwerte (52, 53, 60, 61, 62, 63) für alle eingestellten Scanpositionen (18, 19, 20, 21) der scharfen Ebene ermittelt wird, wobei anschließend ein Maximalwert dieses Verlaufs bestimmt wird, der die Tiefeninformation der Objektoberfläche für diesen Messpunkt des Objekts darstellt.

## Claims

1. A device for the optical 3D measurement of an object (2) using an optical depth-scanning measurement method, comprising at least two light sources (3, 4), at least two optical means (5, 6) for generating structured patterns (30, 34) and at least one recording means (7), wherein a first pattern (30) is generated by means of a first optical means (5) and is projected as a first projection beam (13) onto the object (2) to be recorded, wherein a second pattern (14) is generated by means of a second optical means (6) and is projected onto the object (2) to be recorded as a second projection beam (14), wherein the first pattern (30) and the second pattern (34) are reflected from the object (2) as observation beams (22) and are recorded by means of the recording means (7), to generate a 3D data record of the object, wherein the device (1) features imaging optics (8) for projecting the patterns (30, 34) onto the object (2), wherein the imaging optics (8) are controlled and adjusted such that a sharp focal plane (18) is changed stepwise along an optical axis (24) of the device, wherein at each scan position (18, 19, 20, 21) of the sharp focal plane, the first pattern (30) is projected in order to generate a first optical image, and subsequently at least the second pattern (34) is projected onto the object (2) in order to generate a second optical image, wherein
the device is designed such that the first light source (3) and the second light source (4) are switched on alternately by means of a controller (23), wherein the first pattern (30) and the second pattern (34) are alternately projected onto the object (2), wherein the first projection beam (13) of the first pattern (30) and the second projection beam (14) of the second pattern (34) are deflected by means of a first beam splitter (9) in a common direction of illumination (15) towards the object (2), wherein the projected patterns (30, 34) have the shape of a chessboard with square pattern elements (31, 32) or the shape of parallel stripes (41, 42), wherein the optical depth-scanning measurement method is a depth-from-defocus measurement method, DFD, wherein light pattern elements (32) and dark pattern elements (31) of the two patterns (30, 34) can each be projected alternately onto the object (2) and complement each other, so that a dark pattern element (31) of the second pattern (34) is arranged at a scan position of a light pattern element (32) of the first pattern (30) and vice versa, wherein the optical means which generates the projection pattern is designed as a projection grid, projection mask or digital light projector made of liquid elements, LCD, **characterized in that** depth information of measuring points on the object in a boundary region between the dark and light pattern elements (32) is determined by determining contrast values of the projected pattern (30, 34) and the distance of a sharp focal plane relative to the measuring points on the surface of the object is determined on the basis of these contrast values, wherein a difference of intensity values in the first image and in the second image is determined to determine the contrast values of the measuring points.

2. The device according to claim 1, **characterized in that** the device has a control system which switches the light sources on and off alternately at a frequency of at least 3,000 Hz.

3. The device according to claim 2, **characterized in that** the frequency is advantageously between 5,000 Hz and 10,000 Hz.

4. The device according to any one of claims 1 to 3, **characterized in that** at least 100 scan positions (18, 19, 20, 21) of the sharp focal plane are set and measured in steps to generate the optical image or a scan.

5. The device according to claim 4, **characterized in that** at least 200 scan positions (18, 19, 20, 21) of the sharp focal plane are set per image.

6. The device according to any one of claims 1 to 5, **characterized in that** contrast values between the light and the dark elements (31, 32) of the patterns (30, 34) are determined for each optical image, wherein 3D depth information of the object (2) is determined on the basis of the contrast values (52, 53, 60, 61, 62, 63).

7. The device according to claim 6, **characterized in that** for each measuring point of the object, a course of the contrast values (52, 53, 60, 61, 62, 63) is determined for all set scan positions (18, 19, 20, 21) of the sharp plane, a maximum value of this course being subsequently determined, which represents the depth information of the object surface for this measuring point of the object.

## Revendications

1. Dispositif pour la mesure optique en 3D d'un objet (2) en utilisant une méthode de mesure optique par balayage en profondeur, comprenant au moins deux sources de lumière (3, 4), au moins deux moyens optiques (5, 6) destinés à générer des motifs (30, 34) structurés et au moins un moyen de captage (7), dans lequel un premier motif (30) est généré à l'aide d'un premier moyen optique (5) et est projeté sur l'objet (2) à capter sous la forme d'un premier faisceau de projection (13), dans lequel un deuxième motif (14) est généré au moyen d'un deuxième moyen optique (6) et est projeté sur l'objet (2) à capter sous la forme d'un deuxième faisceau de projection (14), dans lequel le premier motif (30) et le deuxième motif (34) sont réfléchis par l'objet (2) en tant que faisceaux d'observation (22) et sont captés par le moyen de captage (7) afin de créer un ensemble de données en 3D de l'objet, dans lequel le dispositif (1) présente une optique de reproduction (8) destinée à projeter les motifs (30, 34) sur l'objet (2), dans laquelle l'optique de reproduction (8) est commandée et ajustée de telle sorte qu'un plan focal (18) net est modifié pas à pas le long d'un axe optique (24) du dispositif, dans lequel, à chaque position de balayage (18, 19, 20, 21) du plan focal net, le premier motif (30) est projeté pour produire une première prise de vue optique, puis au moins le deuxième motif (34) est projeté sur l'objet (2) pour produire une deuxième prise de vue optique, dans lequel
le dispositif est conçu de telle sorte que la première source de lumière (3) et la deuxième source de lumière (4) sont activées en alternance au moyen d'une commande (23), dans lequel le premier motif (30) et le deuxième motif (34) sont projetés en alternance sur l'objet (2), dans lequel le premier faisceau de projection (13) du premier motif (30) et le deuxième faisceau de projection (14) du deuxième motif (34) sont déviés dans une direction d'éclairage (15) commune vers l'objet (2) au moyen d'un premier diviseur de faisceau (9), dans lequel les motifs (30, 34) projetés présentent la forme d'un damier avec des éléments de motif (31, 32) carrés ou la forme de bandes (41, 42) parallèles, dans lequel la méthode de mesure optique à balayage en profondeur est un procédé de mesure Depth-from-Defocus, DFD, dans lequel des éléments de motif clairs (32) et des éléments de motif sombres (31) des deux motifs (30, 34) peuvent être projetés respectivement en alternance sur l'objet (2) et se complètent, de telle sorte qu'au niveau d'une position de balayage d'un élément de motif clair (32) du premier motif (30) est disposé un élément de motif sombre (31) du deuxième motif (34) et inversement, dans lequel le moyen optique qui génère le motif de projection est réalisé sous la forme d'une grille de projection, d'un masque de projection ou d'un projecteur de lumière numérique constitué d'éléments liquides, LCD, **caractérisé en ce que** des informations de profondeur de points de mesure sur l'objet dans une zone limite entre les éléments de motif sombres et clairs (32) sont déterminées en déterminant des valeurs de contraste du motif (30, 34) projeté et en déterminant à l'aide de ces valeurs de contraste la distance d'un plan focal net par rapport aux points de mesure sur la surface de l'objet, dans lequel, pour définir les valeurs de contraste des points de mesure, on définit une différence de valeurs d'intensité dans la première prise de vue et dans la deuxième prise de vue.

2. Dispositif selon la revendication 1, **caractérisé en ce que** le dispositif présente une commande qui active et désactive en alternance les sources de lumière à une fréquence d'au moins 3 000 Hz.

3. Dispositif selon la revendication 2, **caractérisé en ce que** la fréquence est avantageusement comprise entre 5 000 Hz et 10 000 Hz.

4. Dispositif selon l'une quelconque des revendications 1 à 3, **caractérisé en ce que**, pour générer la prise de vue optique ou un balayage, au moins 100 positions de balayage (18, 19, 20, 21) du plan focal net sont configurées et mesurées pas à pas.

5. Dispositif selon la revendication 4, **caractérisé en ce que**, pour chaque prise de vue, au moins 200 positions de balayage (18, 19, 20, 21) du plan focal net sont configurées.

6. Dispositif selon l'une quelconque des revendications 1 à 5, **caractérisé en ce que** des valeurs de contraste entre les éléments clairs et sombres (31, 32) des motifs (30, 34) sont définies pour chaque prise de vue optique, dans lequel des informations de profondeur en 3D de l'objet (2) sont définies à l'aide des valeurs de contraste (52, 53, 60, 61, 62, 63).

7. Dispositif selon la revendication 6, **caractérisé en ce que**, pour chaque point de mesure de l'objet, on détermine une courbe des valeurs de contraste (52, 53, 60, 61, 62, 63) pour toutes les positions de balayage (18, 19, 20, 21) configurées du plan focal net, puis dans lequel on définit une valeur maximale de cette courbe, laquelle représente l'information de profondeur de la surface de l'objet pour ce point de mesure de l'objet.
